# EUROPÄISCHE PATENTSCHRIFT

(11) **EP 3 501 560 B1**
(45) Veröffentlichungstag und Bekanntmachung des Hinweises auf die Patenterteilung: **18.11.2020**
(21) Anmeldenummer: 18211990.9
(22) Anmeldetag: 12.12.2018
(51) Int. Cl.: A61M 1/00

(54) **VERFAHREN ZUM BETREIBEN EINES UNTERDRUCKTHERAPIESYSTEMS**
METHOD FOR OPERATING A SUCTION THERAPY SYSTEM
PROCÉDÉ DE FONCTIONNEMENT D'UN SYSTÈME DE TRAITEMENT PAR SOUS-PRESSION

(30) Priorität: 21.12.2017 DE 102017131018
(43) Veröffentlichungstag der Anmeldung: 26.06.2019
(73) Patentinhaber: Paul Hartmann AG, 89522 Heidenheim (DE)
(72) Erfinder: Beyrle, Karina, 89075 Ulm (DE); Kunath, Jens, 06785 Oranienbaum-Wörlitz (DE); Partzsch, Christoph, 06846 Dessau-Rosslau (DE); Murgulescu, Mihai, 90411 Nürnberg (DE)
(74) Vertreter: DREISS Patentanwälte PartG mbB

(56) Entgegenhaltungen:
- WO-A1-2016/018448
- WO-A2-2010/017484
- US-A1- 2017 348 469

## Beschreibung

Die Erfindung wird durch die Merkmale des unabhängigen Anspruchs 1 definiert.

Vorrichtungen zur Unterdruckwundbehandlung und Verfahren zum Betreiben der Vorrichtungen sind bereits mehrfach beschrieben worden, insbesondere durch WO 2012/156140 A1 und WO 2008/100440 A1.

Bei derartigen Vorrichtungen zur Unterdruckbehandlung von Wunden kommuniziert eine Saugpumpe über eine Saugleitung mit der Wunde oder der Wundumgebung, wobei ein Wundverband mit einem luftundurchlässigen Abdeckmaterial zum luftdichten Verschließen der Wunde und der Wundumgebung vorgesehen ist, so dass ein Unterdruck im Wundraum herstellbar und Flüssigkeiten aus dem Wundraum in den genannten Behälter absaugbar sind.

Neben der Unterdruckwundbehandlung sind auch andere Anwendungen der hier in Rede stehenden Vorrichtung zur Bereitstellung von Unterdruck für medizinische Anwendungen denkbar, insbesondere die Absaugung an sich beliebiger Körperflüssigkeiten, im Bereich der medizinischen Inkontinentenversorgung, der Versorgung von Stoma-Patienten oder im Bereich der Absaugung von Wundsekreten, gegebenenfalls unter Verwendung von Spülflüssigkeiten, auch ohne Anlegen eines Unterdrucks über wesentliche Zeiträume.

Bei dem in Rede stehenden Unterdrucktherapiesystem handelt es sich vorzugsweise um eine tragbare Vorrichtung. Das bedeutet, dass der Patient das Unterdrucktherapiesystem mitführen kann, so dass er mobil ist und dennoch seine Wunde dauerhaft, d.h. ohne Unterbrechung, therapiert werden kann. Die tragbare Vorrichtung kann dabei über an sich beliebige Befestigungsmittel, insbesondere und vorzugsweise in Form eines flexiblen Gürtels oder eines Schultertragriemens, am Körper des Patienten gehalten und mitgeführt werden. Das Unterdrucktherapiesystem der hier in Rede stehenden Art kann natürlich auch im stationären Betrieb, also losgelöst vom Körper des Patienten, eingesetzt werden; es kann solchenfalls beispielsweise an einem Pflegebett befestigt oder neben dem Pflegebett abgestellt werden.

Der Begriff eines Unterdrucks bezeichnet im Zusammenhang mit der vorliegenden Erfindung einen gegenüber dem Umgebungsluftdruck (atmosphärischer Luftdruck) erniedrigten Luftdruck insbesondere innerhalb eines Wundverbands. Das Abdeckmaterial eines Wundverbands zum luftdichten Verschließen eines Wundraums muss daher so ausgebildet sein, dass es der sich einstellenden Druckdifferenz standzuhalten vermag, damit der Unterdruck im Wundraum überhaupt angelegt und aufrechterhalten werden kann. Eine gewisse Nachgiebigkeit des Wundverbands und Abdeckmaterials ist jedoch typischerweise gegeben. Im Bereich der Unterdrucktherapie in der Wundbehandlung wird der Unterdruck quantitativ als Druckdifferenz zwischen dem Umgebungsluftdruck und dem unterhalb des Abdeckmaterials angelegten Luftdruck angegeben. Typischerweise beträgt diese Druckdifferenz im Bereich der Unterdrucktherapie höchstens 250 mmHg (mm Quecksilbersäule) (1 mmHg = 1 Torr. = 133,322 Pa). Dieser Unterdruckbereich bis höchstens 250 mmHg hat sich als für die Wundheilung geeignet erwiesen. Ein bevorzugter Unterdruckbereich liegt zwischen 10 und 150 mmHg.

Der unter Verwendung der Vorrichtung an die Wunde angelegte Unterdruck kann bei einer typischen Unterdruckbehandlung entweder zeitlich im Wesentlichen konstant gehalten werden, oder er kann zeitlich verändert, insbesondere zyklisch verändert werden, was über eine entsprechend ausgebildete und programmierte elektronische Steuereinrichtung bei dem Unterdrucktherapiesystem, insbesondere in Abhängigkeit weiterer Parameter, realisiert werden kann. Insbesondere ist der an der Wunde angelegte Unterdruck auf einen vorgebbaren Zielunterdruck einregelbar.

Zum Anlegen von Unterdruck und vorzugsweise auch zum Absaugen von Körperflüssigkeiten ist eine vorzugsweise flexible Saugleitung, beispielsweise in Form eines Drainageschlauchs, vorgesehen, der einenends über einen sogenannten Port im Bereich des Wundabdeckmaterials mit der Wundumgebung oder dem Wundraum und anderenends mit dem eingangs erwähnten Behälter zur Aufnahme von Körperflüssigkeiten bzw. mit der Unterdruck erzeugenden Einrichtung kommuniziert.

Zum Erfassen des Drucks im Unterdrucktherapiesystem ist ein Drucksensor vorgesehen. Der Drucksensor kann in grundsätzlich vielfältiger Weise realisiert und in dem Unterdrucktherapiesystem vorgesehen sein. Der Drucksensor kann zur Messung des Drucks beispielsweise in einer zum Körper führenden Druckmessleitung oder in der Saugleitung oder im Behälter oder im Wundraum ausgebildet und angeordnet sein. Typischerweise ist nur ein einziger Drucksensor für das Unterdrucktherapiesystem vorgesehen.

Der von dem Drucksensor erfasst Wert wird an die elektronische Steuereinrichtung weitergegeben und von dieser verarbeitet und löst im weitesten Sinne weitere Steuerungsprozesse aus, nämlich zumindest die Aktivierung und/oder Deaktivierung der Saugpumpe.

Zur Verwendung des Unterdrucktherapiesystems wird die Saugpumpe aktiviert, wenn kein Unterdruck vorliegt oder der mittels des Drucksensors erfasste Unterdruck niedriger als der vorgegebene Zielunterdruck ist.

Wenn der Zielunterdruck erreicht ist, wird die Saugpumpe deaktiviert. Typischerweise gilt der Zielunterdruck als erreicht, wenn der Unterdruck nur noch um eine vorgebbare beziehungsweise vorgegebene Toleranz, insbesondere +/- 25%, von dem vorgegebenen Zielunterdruck abweicht.

Wenn in diesem Sinne von Deaktivieren der Saugpumpe die Rede ist, so wird hierunter verstanden, dass der gerade ablaufende Druckregelbetrieb und damit die Ansteuerung der Pumpe zumindest vorübergehend unterbrochen werden. Es ist jedoch vorgesehen, dass die Saugpumpe zum Aufrechterhalten des Unterdrucks oder zum erneuten Aufbau von Unterdruck vorübergehend erneut aktiviert wird.

Beim Betreiben eines Unterdrucktherapiesystems ist es problematisch, wenn Fehler auftreten, die eine korrekte Funktion des Unterdrucktherapiesystems einschränken. Beispielsweise können Leckagen und/oder Blockaden dazu führen, dass eine korrekte Unterdruckbehandlung der Wunde nicht mehr gewährleistet werden kann. Zum Erkennen solcher Fehler sind bereits einige Verfahren bekannt.

Aus der WO 2008/100440 A1 ist beispielsweise ein Unterdrucktherapiesystem mit einer Druckmessleitung bekannt, bei dem Leckagen und Blockaden über eine Geschwindigkeit der Pumpe erfasst werden. Die WO 2008/103406 A1 offenbart ein Unterdrucktherapiesystem, bei dem eine Leckage über Druckwerte und Pumpenleistungswerte ermittelt wird. Schließlich offenbart die WO 2010/017484 A2 ein Unterdrucktherapiesystem, bei dem ein Kanister-voll-Zustand anhand von Druckwerten und Leistungswerten der Pumpe erkannt werden kann. Folgende sind weitere relevante Stände der Technik: WO 2016/018448 A1, US 2017/348469 A1.

Der vorliegenden Erfindung liegt die Aufgabe zugrunde, ein Unterdrucktherapiesystem bereitzustellen, mit dem Blockaden und Leckagen zuverlässig detektiert und voneinander unterschieden werden können.

Zur Lösung dieser Aufgabe wird bei einem Unterdrucktherapiesystem der genannten Art erfindungsgemäß vorgeschlagen, dass bei aktivierter Saugpumpe geprüft wird, ob innerhalb einer vorgegebenen ersten Zeitspanne Δt1 eine Druckänderungsrate in Richtung zunehmenden Unterdrucks unterhalb eines vorgegebenen ersten Schwellenwerts S1 bleibt, und dass geprüft wird, ob ein Unterdruck oberhalb eines vorgegebenen zweiten Schwellenwerts S2 vorliegt, und sofern beides zutrifft, die Saugpumpe gestoppt wird, und dass bei gestoppter Saugpumpe die Druckänderungsrate in Richtung abnehmenden Unterdrucks ermittelt wird, und dass auf eine Leckage im Unterdrucktherapiesystem geschlossen wird, wenn die Druckänderungsrate oberhalb eines vorgegebenen dritten Schwellenwerts S3 liegt, und/oder dass auf eine Blockade im Unterdrucktherapiesystem geschlossen wird, wenn die Druckänderungsrate unterhalb eines vorgegebenen vierten Schwellenwerts S4 liegt.

Bei aktivierter Saugpumpe wird also zum einen die Druckänderungsrate ermittelt. Die Druckänderungsrate in ΔP/Δt beschreibt das Ausmaß der Veränderung des Druckes P in einem bestimmten Zeitraum im Verhältnis zur Dauer des Zeitraums.

Weiter wird vorgeschlagen, dass die folgenden Prüfungen durchgeführt werden:
a) Bleibt die Druckänderungsrate in Richtung zunehmenden Unterdrucks innerhalb der vorgegebenen ersten Zeitspanne Δt1 unterhalb eines vorgegebenen ersten Schwellenwerts S1?
b) Liegt ein Unterdruck oberhalb eines vorgegebenen zweiten Schwellenwerts S2 vor?

Für den Fall, dass a) und b) zutreffen, wird die Saugpumpe gestoppt und die Druckänderungsrate in Richtung abnehmenden Unterdrucks wird ermittelt und die folgende Prüfung durchgeführt:
c) Liegt die Druckänderungsrate oberhalb des vorgegebenen dritten Schwellenwerts S3?

Wenn die Druckänderungsrate oberhalb des vorgegebenen dritten Schwellenwerts S3 liegt, also wenn c) zutrifft, wird auf eine Leckage im Unterdrucktherapiesystem geschlossen.

Andernfalls, wenn die Druckänderungsrate nicht oberhalb des vorgegebenen dritten Schwellenwertes S3 liegt, wird geprüft:
d) Liegt die Druckänderungsrate unterhalb des vorgegebenen vierten Schwellenwerts S4?

Wenn die Druckänderungsrate unterhalb des vorgegebenen vierten Schwellenwerts S4 liegt, also wenn d) zutrifft, wird auf eine Blockade im Unterdrucktherapiesystem geschlossen.

Nach einer Ausführungsform der Erfindung beträgt die erwähnte vorgegebene erste Zeitspanne Δt1 0,5s bis 2s, vorzugsweise 0,8s bis 1,3s, insbesondere 1s. Es wird also geprüft, ob innerhalb dieser ersten vorgegebenen Zeitspanne Δt1, eine Druckänderungsrate in Richtung zunehmenden Unterdrucks unterhalb eines vorgegebenen ersten Schwellenwerts S1 bleibt.

Der erwähnte erste Schwellenwert S1 für die Druckänderungsrate beträgt vorzugsweise 0 mmHg/s. Die Druckänderungsrate liegt also unterhalb des Schwellenwerts S1, wenn der Druck konstant bleibt oder wenn der Unterdruck abnimmt. Mit anderen Worten, der Schwellenwert S1 wird also überschritten, wenn eine Druckänderung in Richtung zunehmenden Unterdrucks festgestellt wird. S1 kann aber auch einen geringen positiven Schwellenwert haben.

Der erwähnte zweite Schwellenwert S2 für den Unterdruck beträgt vorteilhafterweise 10 mmHg bis 25 mmHg, vorzugweise 10 mmHg bis 20 mmHg, insbesondere 15 mmHg.

Vorteilhafterweise beträgt der erwähnte dritte Schwellenwert S3 5 mmHg/15 s bis 15 mmHg/15 s, insbesondere 8 mmHg/15 s, und/oder der erwähnte vierte Schwellenwert S4 beträgt 1 mmHg/15 s bis 5 mmHg/15 s, insbesondere 2 mmHg/15 s. Typischerweise ist der dritte Schwellenwert S3 zumindest genauso groß wie der vierte Schwellenwert S4. Insbesondere ist der dritte Schwellenwert S3 jedoch größer als der vierte Schwellenwert S4.

Für den Fall, dass die oben genannten Prüfungen a) und b) zutreffen, wird die Saugpumpe gestoppt und die Druckänderungsrate in Richtung abnehmenden Unterdrucks wird ermittelt. Hierbei erweist es sich als vorteilhaft, dass die Saugpumpe für eine Dauer von 5 s bis 30 s, vorzugsweise 10 s bis 20 s, insbesondere 15 s gestoppt wird.

Gemäß einer bevorzugten Weiterbildung der Erfindung wird vorgeschlagen, dass geprüft wird, ob innerhalb der vorgegebenen ersten Zeitspanne Δt1 eine Druckänderungsrate in Richtung zunehmenden Unterdrucks unterhalb des vorgegebenen ersten Schwellenwerts S1 bleibt, und dass geprüft wird, ob ein Unterdruck oberhalb des vorgegebenen zweiten Schwellenwerts S2 vorliegt, und sofern ersteres zutrifft und zweiteres nicht zutrifft, eine elektrische Leistung oder Leistungsaufnahme des Unterdrucktherapiesystems ermittelt wird, und dass auf eine Leckage im Unterdrucktherapiesystem geschlossen wird, wenn die elektrische Leistung oder Leistungsaufnahme unterhalb eines vorgegebenen fünften Schwellenwerts S5 liegt, und/oder dass auf eine Blockade im Unterdrucktherapiesystem geschlossen wird, wenn die elektrische Leistung oder Leistungsaufnahme oberhalb des vorgegebenen fünften Schwellenwerts S5 liegt.

Es wird also, wie vorstehend bereits beschrieben, vorgeschlagen, dass die folgenden Prüfungen a) und b) durchgeführt werden:
a) Bleibt die Druckänderungsrate in Richtung zunehmenden Unterdrucks innerhalb der vorgegebenen ersten Zeitspanne Δt1 unterhalb eines vorgegebenen ersten Schwellenwerts S1?
b) Liegt ein Unterdruck oberhalb eines vorgegebenen zweiten Schwellenwerts S2 vor?

Für den Fall, dass a) zutrifft, also die Druckänderungsrate in Richtung zunehmenden Unterdrucks innerhalb der vorgegebenen ersten Zeitspanne Δt1 unterhalb des vorgegebenen ersten Schwellenwerts S1 liegt, und b) nicht zutrifft, also der Unterdruck nicht oberhalb des vorgegebenen zweiten Schwellenwerts S2 liegt, sondern mit anderen Worten, im Wesentlichen Atmosphärendruck vorliegt, dann wird die elektrische Leistung oder Leistungsaufnahme des Unterdrucktherapiesystems ermittelt und folgende Prüfung durchgeführt:
e) Liegt die elektrische Leistung unterhalb oder oberhalb eines vorgegebenen fünften Schwellenwerts S5?

Nach diesem weiteren Gedanken wird also bei aktivierter Saugpumpe und wenn die Druckänderungsrate innerhalb der vorgegeben ersten Zeitspanne Δt1 unterhalb des vorgegebenen ersten Schwellenwerts S1 liegt, also wenn sich der Unterdruck gar nicht oder nur mit einer Druckänderungsrate unterhalb des Schwellenwerts S1 aufbaut, unterschieden, ob Unterdruck oberhalb oder unterhalb des vorgegebenen zweiten Schwellenwerts S2 vorliegt. Und für den Fall, dass der Unterdruck nicht oberhalb des zweiten Schwellenwerts vorliegt, insbesondere, wenn also im Wesentlichen Atmosphärendruck vorliegt, erfolgt das Schließen auf eine Leckage und/oder eine Blockade in Abhängigkeit der elektrischen Leistung des Unterdrucktherapiesystems und nicht mehr in Abhängigkeit der Druckänderungsrate bei ausgeschalteter Saugpumpe.

Für den Fall, dass die elektrische Leistung unterhalb des fünften Schwellenwerts S5 liegt, wird auf eine Leckage im Unterdrucktherapiesystem geschlossen. Für den Fall, dass die elektrische Leistung oberhalb des vorgegebenen fünften Schwellenwerts S5 liegt, wird auf eine Blockade im Unterdrucktherapiesystem geschlossen.

Bei der elektrischen Leistung des Unterdrucktherapiesystems kann es sich insbesondere um eine aufgenommene elektrische Leistung des Unterdrucktherapiesystems oder um eine aufgenommene elektrische Leistung der Saugpumpe handeln. Bei dem Schwellenwert S5 kann es sich um einen vorgegebenen konstanten, insbesondere in die elektronische Steuereinrichtung implementierten, Wert handeln. Es ist aber auch denkbar, dass der Schwellenwert S5 vor der Verwendung oder zu Beginn der Verwendung des Unterdrucktherapiesystems in einem Kalibrierverfahren festgelegt wird. Beispielsweise kann das Bestimmen des fünften Schwellenwerts S5 über die Ermittlung der Leistungsaufnahme des Geräts in einem Leckagezustand bei einer Ansteuerung der Pumpe mit Pulsweitenmodulation (PWM) mit einem festgelegten Ansteuertaktverhältnis, insbesondere 50%, erfolgen, in dem über einem definierten Widerstand die Spannung gemessen und daraus ein durch das Gerät fliesender Strom bestimmt wird, wobei die Berechnung der Leistungsaufnahme unter Einbeziehung einer Batteriespannung des Unterdrucktherapiesystems erfolgt. Der Leckagezustand kann durch ein Lösen des Saugschlauches oder des Behälters von dem Gerät erzeugt werden.

Typische Kalibrierungswerte sind beispielsweise 166.0 mW und 171.6 mW.

Der vorliegenden Erfindung kommt in besonderem Maße Bedeutung beim mobilen Einsatz von Unterdrucktherapiesystemen der hier in Rede stehenden Art zu, da mit einem solchen tragbaren Unterdrucktherapiesystem ausgestattete Patienten fernab von einer klinischen Einrichtung auf sich selbst gestellt sind, und ein Fehlerzustand, insbesondere in Form einer Leckage und/oder Blockade und/oder eine damit einhergehende Beeinträchtigung der Funktion des Unterdrucktherapiesystems zuverlässig erfasst und dem Patienten angezeigt werden soll.
In bevorzugter Weiterbildung der Erfindung erweist es sich daher als vorteilhaft, wenn bei Schließen auf eine Leckage und/oder bei Schließen auf eine Blockade ein Signal ausgegeben wird. Bei dem Signal kann es sich beispielsweise um ein akustisches und/oder ein visuelles Signal handeln. Es ist denkbar, dass das Signal unmittelbar oder zeitlich verzögert bei Schließen auf eine Leckage und/oder bei Schließen auf eine Blockade ausgegeben wird.

In Weiterbildung der Erfindung erweist es sich als vorteilhaft, wenn bei Schließen auf eine Leckage und/oder bei Schließen auf eine Blockade die Verfahrensschritte gemäß der Ansprüche 1 und/oder 7 wenigstens einmal oder mehrere Male erneut ausgeführt werden, bevor definitiv auf eine Leckage oder Blockade geschlossen wird. Vorzugsweise werden die Verfahrensschritte der Ansprüche 1 und 7 bei erstmaligen Schließen auf eine Leckage und/oder bei erstmaligem Schließen auf eine Blockade wenigstens einmal oder mehrere Male erneut durchgeführt, bevor definitiv auf eine Leckage oder eine Blockade geschlossen wird und/oder das Signal erneut ausgegeben wird.

Im Normalbetrieb des Unterdrucktherapiesystems wird die Saugpumpe aktiviert, wenn der vorliegende Unterdruck von dem Zielunterdruck abweicht. Sie wird deaktiviert, wenn der Zielunterdruck erreicht ist. Nun kann es im laufenden Betrieb in einem ungünstigen Fall dazu kommen, dass zwar ein Unterdruckabfall stattfindet, aber dieser von dem Drucksensor nicht erkannt wird. Dies ist beispielsweise der Fall, wenn der Unterdruck in der Druckmessleitung infolge einer Blockade aufrechterhalten bleibt, in der Saugleitung und/oder im Behälter jedoch abfällt. In bevorzugter Weiterbildung der Erfindung erweist sich die Ausführung einer Blockadeerkennung als vorteilhaft. Hierfür wird vorgeschlagen, dass geprüft wird, ob der vorgegebene Zielunterdruck vorliegt, und dass geprüft wird, ob die Saugpumpe seit mindestens einer vorgegebenen zweiten Zeitspanne Δt2 deaktiviert ist, und sofern beides zutrifft, die Saugpumpe für eine vorgegebene dritte Zeitspanne Δt3 aktiviert wird und anschließend bei deaktivierter Saugpumpe über eine Zeitspanne Δt4 eine maximale Druckänderung in Richtung zunehmenden Unterdrucks und/oder eine maximale Druckänderung in Richtung abnehmenden Unterdrucks ermittelt und geprüft wird, ob die maximale Druckänderung in Richtung zunehmenden Unterdrucks oberhalb eines vorgegebenen sechsten Schwellenwerts S6 und/oder die maximale Druckänderung in Richtung abnehmenden Unterdrucks oberhalb eines vorgegeben siebten Schwellenwerts S7 liegt.

Die maximale Druckänderung in Richtung zunehmenden oder abnehmenden Unterdrucks kann als Differenz des jeweiligen Extremwerts für den Unterdruck während der Zeitspanne Δt4 und des Werts des Unterdrucks zu Beginn der Zeitspanne Δt4 ermittelt werden.

Die vorgegebene zweite Zeitspanne Δt2 beträgt vorteilhafterweise 5 min bis 15 min, vorzugsweise 8 min bis 12 min, insbesondere 10 min. Nach einer bevorzugten Ausführungsform wird die Zeitspanne Δt2 in Abhängigkeit von einem Verzögerungsfaktor berechnet, wobei der Verzögerungsfaktor wiederum davon abhängt, wie lange bereits der Zielunterdruck im System vorliegt.

Die vorgegebene dritte Zeitspanne Δt3 beträgt vorteilhafterweise 100 ms bis 500 ms, vorzugsweise 200 ms bis 300 ms. Nach einer bevorzugten Ausführungsform ist die dritte Zeitspanne Δt3 von dem vorgegebenen Zielunterdruck abhängig.

Es wird also vorgeschlagen, dass die Saugpumpe, sofern am Drucksensor Zielunterdruck vorliegt und die Saugpumpe seit mindestens der zweiten Zeitspanne Δt2 deaktiviert ist, für eine kurze Zeitspanne Δt3 aktiviert wird. Anschließend wird bei deaktivierter Pumpe über eine Zeitdauer Δt4 eine maximale Druckänderung in Richtung zunehmenden und/oder in Richtung abnehmenden Unterdrucks, die durch das kurzzeitige Aktivieren der Saugpumpe hervorgerufen wird, ermittelt und überprüft, ob die maximale Druckänderung in Richtung zunehmenden Unterdrucks oberhalb eines vorgegeben sechsten Schwellenwerts S6 und/oder die maximale Druckänderung in Richtung abnehmenden Unterdrucks oberhalb eines vorgegeben siebten Schwellenwerts S7 liegt.

Die Zeitdauer Δt4 beträgt vorteilhafterweise 1 min bis 3 min, insbesondere 2 min. Der sechste Schwellenwert S6 beträgt vorteilhafterweise 0,1 mmHg bis 0,3 mmHg, insbesondere 0,2 mmHg. Der siebte Schwellenwert S7 beträgt vorteilhafterweise 3 mmHg bis 4 mmHg, insbesondere 3,5 mmHg.

Für den Fall, dass die maximale Druckänderung in Richtung zunehmenden Unterdrucks oberhalb eines vorgegebenen sechsten Schwellenwerts S6 und/oder die maximale Druckänderung in Richtung abnehmenden Unterdrucks oberhalb eines vorgegebenen siebten Schwellenwerts S7 liegt, wird darauf geschlossen, dass kein Fehler in Form einer Blockade vorliegt sondern die Leitungsmittel jedenfalls durchgängig sind. Wenn die maximale Druckänderung in Richtung zunehmenden oder abnehmenden Unterdrucks unterhalb des jeweiligen Schwellenwerts liegt, wird auf eine Blockade geschlossen. Der Verlauf der vom Drucksensor gemessenen Druckänderung während der Zeitspanne Δt4 kann sehr verschieden ausfallen. Bei einem trockenen durchgängigen, also nicht blockierten System wird die Druckänderung sehr schnell am Drucksensor ankommen und messbar sein, d.h. hier kann es in den ersten 10s von Δt4 eine deutliche Druckänderung geben, wobei danach der Druck dann ziemlich konstant bleibt, sofern das System dicht ist. Bei einem System mit sehr viel Flüssigkeit in den Schläuchen dauert es deutlich länger, ehe eine geringe Druckänderung des vorstehenden Blockadeerkennungs-Tests am Drucksensor sichtbar wird, daher wird auch die Zeitspanne Δt4 recht lang gewählt.

Es erweist sich weiter als vorteilhaft, wenn das Unterdrucktherapiesystem eine Ausgabe- oder Anzeigeeinrichtung zur Erzeugung eines Signals umfasst, welches das Schließen auf eine Leckage und/oder das Schließen auf eine Blockade visuell oder akustisch vermittelt. Auf diese Weise kann dem Benutzer und/oder dem Patienten angezeigt werden, wenn eine Leckage und/oder eine Blockade im System vorliegt.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der Erfindung. In der Zeichnung zeigt:
- Figur 1: eine bevorzugte Ausführungsform eines erfindungsgemäßen Unterdrucktherapiesystems;
- Figur 2: ein Gehäuse des Unterdrucktherapiesystems nach Figur 1;
- Figur 3: eine schematische Darstellung eines erfindungsgemäßen Unterdrucktherapiesystems an einer Wunde;
- Figur 4: eine schematische Darstellung eines Verfahrens; und
- Figuren 5a bis 5e: eine skizzenhafte Darstellung verschiedener Fehlerzustände, die von einem erfindungsgemäßen Unterdrucktherapiesystem erfasst werden können.

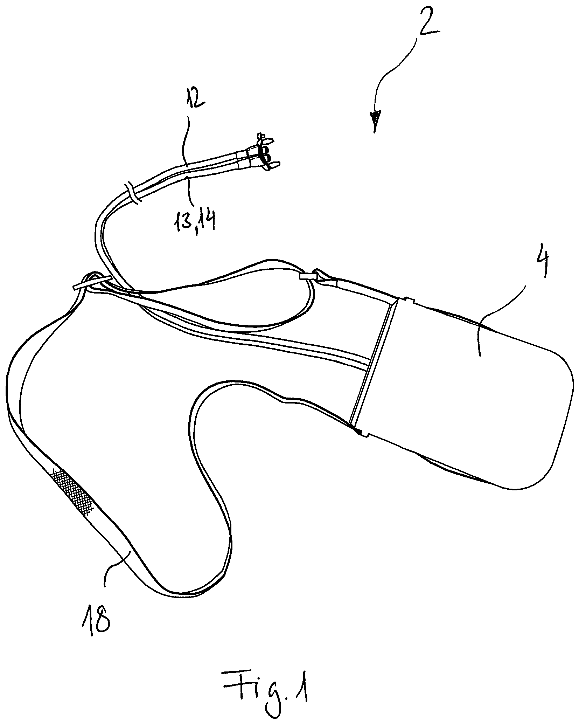

Figur 1 zeigt eine Ausführungsform eines erfindungsgemäßen Unterdrucktherapiesystems zum Bereitstellen von Unterdruck zur medizinischen Unterdruckbehandlung, das in seiner Gesamtheit mit dem Bezugszeichen 2 gekennzeichnet ist. Das Unterdrucktherapiesystem 2 umfasst ein Gehäuse 4, in dem eine Unterdruck erzeugende Saugpumpe 6, ein Behälter 8 zur Aufnahme von Körperflüssigkeiten, insbesondere von aus einer Wunde abgesaugten Wundsekreten und eine elektronische Steuereinrichtung 10 zum Steuern des Unterdrucktherapiesystems 2, insbesondere zum Ansteuern der Saugpumpe 6, angeordnet sind.

Bei der Saugpumpe 6 handelt es sich beispielsweise um eine Membranpumpe.

Des Weiteren umfasst das Unterdrucktherapiesystem 2 eine zum Körper führende Saugleitung 12, die einenends zu einem die Wunde druckdicht verschließen Wundverband führt und dort beispielsweise über einen Port/Anschluss mit dem Wundraum kommuniziert, um im Wundraum einen Unterdruck anzulegen und aufrecht zu erhalten. Anderenends ist die Saugleitung 12 im beispielhaft dargestellten Fall mit dem bereits erwähnten Behälter 8 verbunden, um Wundsekret in den Behälter 8 abzusaugen. Hierfür kommuniziert der Behälter 8 mit der Saugpumpe 6. Weiter ist eine zusätzliche Leitung 13, die wie die Saugleitung 12 zur Wunde führt, dargestellt. Hierbei kann es sich um eine Instillations- oder Spülleitung oder um eine Druckmessleitung 14 handeln.

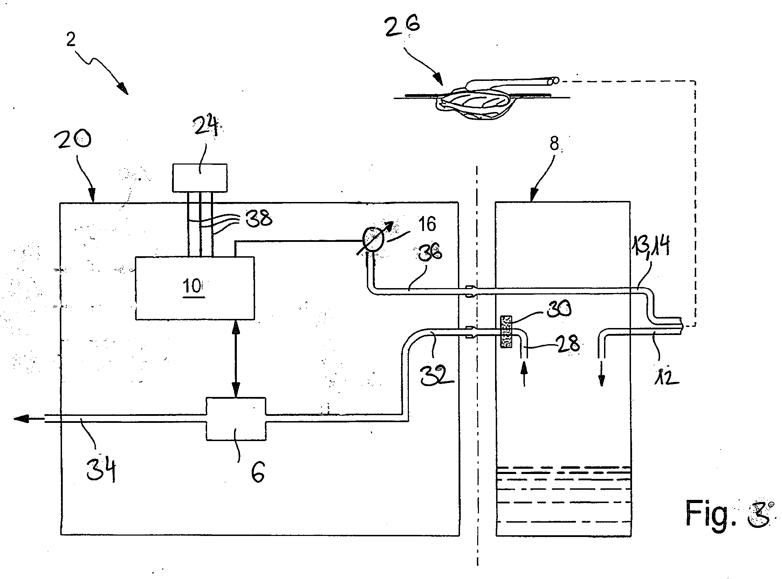

Ferner umfasst das Unterdrucktherapiesystem 2 einen erst in Figur 3 dargestellten Drucksensor 16 zum Erfassen des Drucks in der zum Körper führenden Saugleitung 12 oder in der als Druckmessleitung 14 ausgebildeten zusätzlichen Leitung 13 oder im Behälter 8 oder in der Wunde 26.

Ferner umfasst das Unterdrucktherapiesystem 2 gemäß der beispielhaft dargestellten Ausführungsform ein gurtartiges Haltemittel 18, so dass das System 2 am Körper eines Benutzers tragbar und mitführbar ist.

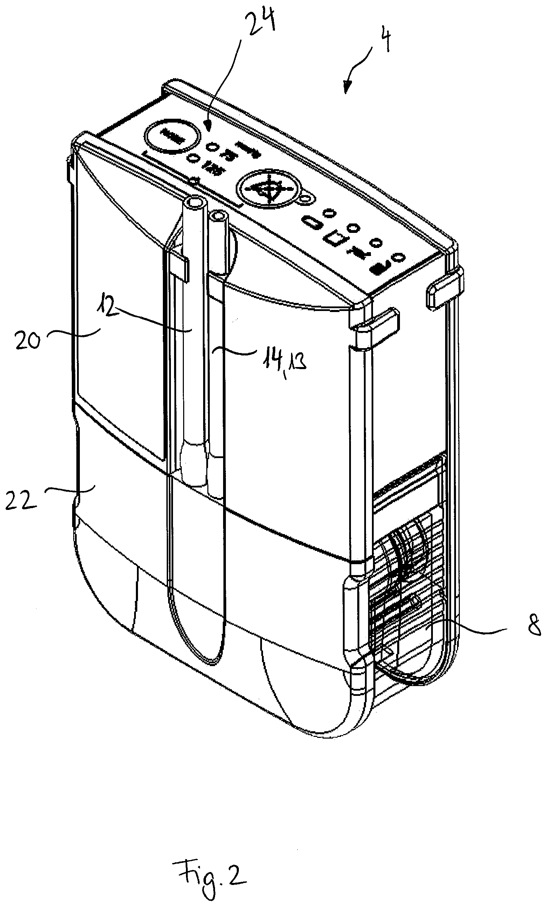

Gemäß der in Figur 2 dargestellten Ausführungsform des Unterdrucktherapiesystems 2 umfasst das Gehäuse 4 zwei lösbar gegeneinander fixierbare Gehäuseteile 20, 22. In dem ersten Gehäuseteil 20 sind die Saugpumpe 6 sowie die elektronische Steuereinrichtung 10 einschließlich Batterien oder vorzugsweise wiederaufladbar Akkus aufgenommen. Der zweite Gehäuseteil 22 bildet im bevorzugten Fall zugleich den Behälter 8 zur Aufnahme der Körperflüssigkeiten. Bevorzugtermaßen ist der gesamte zweite Gehäuseteil 22 als wegwerfbarer Einmalartikel ausgebildet.

Die Saugleitung 12 und die zusätzliche Leitung 13 sind in Figur 2 nur angedeutet. Die zusätzliche Leitung 13 führt durch den zweiten Gehäuseteil 22, also den Behälter 8 hindurch, und mündet in den ersten Gehäuseteil 20, von wo aus beispielsweise ein Druck detektiert und ausgewertet werden kann.

Zur Ausbildung der Unterdruckkommunikation weisen die Gehäuseteile 20, 22 nicht dargestellte Anschluss- und Gegenanschlussmittel auf, über die eine nach außen abgedichtete Druckkommunikation ausbildbar ist.

Des Weiteren umfasst das Unterdrucktherapiesystem 2 Bedien- und Anzeigeelemente 24, die gemäß der dargestellten Ausführungsform an dem ersten Gehäuseteil 20 ausgebildet sind. Über diese Elemente 24 kann das Unterdrucktherapiesystem 2 beispielsweise ein- und ausgeschaltet und/oder ein gewünschter Zielunterdruck vorgegeben werden. Ferner kann über die Anzeigeelemente ein Status des Unterdrucktherapiesystems 2, beispielsweise An- /Aus- Zustand und/oder ein Fehlerzustand, angezeigt werden.

Figur 3 zeigt das vorausgehend beschriebene oder ein ähnliches Unterdrucktherapiesystem 2 in rein schematischer Darstellung, wobei für entsprechende Bauelemente entsprechende Bezugszeichen verwendet werden. In Figur 3 sind jedoch nur die für die nachfolgende Beschreibung der Funktionsweise relevanten Komponenten dargestellt. Man erkennt schematisch eine in Figur 3 lediglich angedeutete mit Unterdruck zu therapierende Wunde 26 mit einer unterdruckdichten Wundauflage, zu der die aus dem Behälter 8 ausmündende Saugleitung 12 führt. Aus dem Behälter führt ein weiterer Leitungsabschnitt 28 der Saugleitung 12 durch einen Filter 30 hindurch nach außen. Wenn der Behälter 8 bzw. der zweite Gehäuseteil 22 an dem Gehäuse 4 oder an dem ersten Gehäuseteil 20 angebracht ist, so wird der Leitungsabschnitt 28 mit einem weiteren Leitungsabschnitt 32 innerhalb des ersten Gehäuseteils 20 gekoppelt, welcher zu einer Ansaugseite der Saugpumpe 6 führt. Im Betrieb der Saugpumpe 6 wird somit über die Leitungsabschnitte 28, 32 ein Unterdruck an den Behälter 8 und an die Saugleitung 12 gelegt, und die von dort angesaugte Luft über eine Ausgleichsleitung 34 an die Umgebung ausgestoßen.

Des Weiteren dargestellt ist die zusätzliche Leitung 13, die im beispielhaft dargestellten Fall durch den Behälter 8 hindurchführt und ebenso wie die Saugleitung 12 zu der Wunde 26 führt. Beim Anbringen des Behälters 8 in seiner bestimmungsgemäßen Montageposition am ersten Gehäuseteil 20 kommuniziert diese Leitung 13 mit einem im ersten Gehäuseteil 20 vorgesehenen Leitungsabschnitt 36. In der dargestellten Ausführung ist die Leitung 13 eine Druckmessleitung 14. Zu diesem Zweck ist der bereits erwähnte Drucksensor 16 zur Messung des Drucks in dem Leitungsabschnitt 36 vorhanden, dessen Signale an die elektronische Steuereinrichtung 10 gegeben werden, welche die Vorrichtung 2 insgesamt steuert bzw. regelt.

Mit Bezugszeichen 24 sind die bereits erwähnten Bedien- und Anzeigeelemente bezeichnet, über welche die Bedienung der Vorrichtung insgesamt ausführbar ist. Die elektrische Verbindung zu der elektronischen Steuereinrichtung 10 ist über elektrische Leitungen 38 nur angedeutet. Durch die elektronische Steuereinrichtung 10 erfolgt die Ansteuerung der Saugpumpe 6, indem unter Verwendung der Signale des Drucksensors 16 eine Druck- bzw. Unterdruckregelung durch an sich bekannte Steuer- und Regelmechanismen (Soll-/Ist-Regelmechanismen) ausgeführt wird, so dass in dem Leitungsabschnitt 36 der dem gerade gewählten Programm entsprechende Druckwert eingeregelt wird.

Zur Verwendung des Unterdrucktherapiesystems 2, also zum Anlegen von Unterdruck und gegebenenfalls auch zum Absaugen von Körperflüssigkeiten, ist die elektronische Steuereinrichtung 10 so ausgebildet, dass die unterdruckerzeugende Saugpumpe 6 aktiviert und angesteuert wird, wenn ein vorgegebener zu erreichender Zielunterdruck noch nicht erreicht ist, und dass die Saugpumpe 6 deaktiviert wird, wenn der vorgegebene Zielunterdruck erreicht ist.

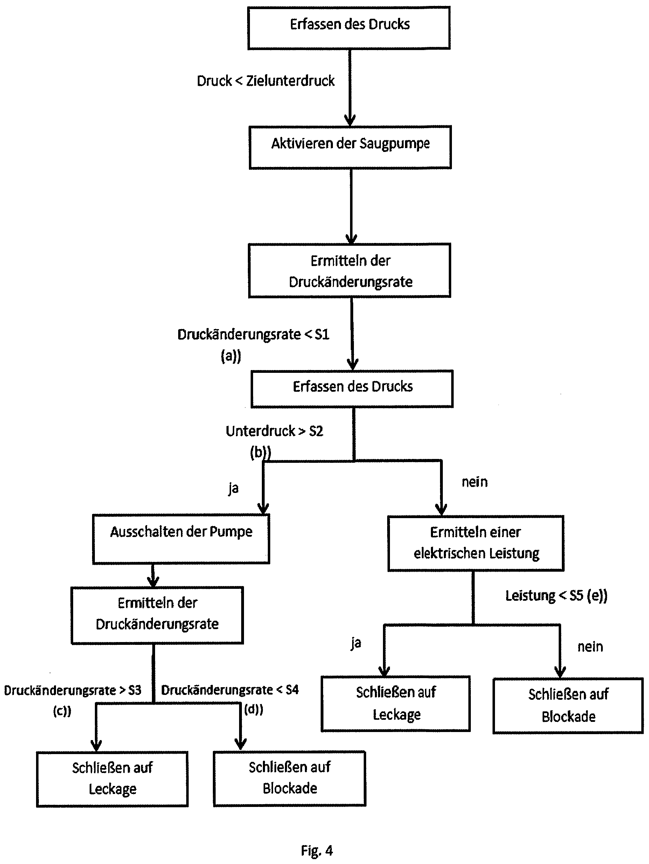

Des Weiteren ist die elektronische Steuereinrichtung zur Ausführung eines Verfahrens zum Erkennen von Leckagen und/oder Blocken ausgebildet. Der Ablauf des Verfahrens ist in Figur 4 schematisch dargestellt. Die Reihenfolge der einzelnen Verfahrensschritte ist dabei beispielhaft, jedoch bevorzugt. Die einzelnen Verfahrensschritte könnten vielmehr auch zeitgleich oder in einer anderen, von der dargestellten zeitlichen Reihenfolge abweichenden Reihenfolge ausgeführt werden.

Zunächst wird, wie schon erwähnt, mittels des Drucksensors 16 ein Druck erfasst und die Saugpumpe 6 aktiviert, um einen vorgegebenen Zielunterdruck zu erreichen, sofern der erfasste Druck unterhalb des vorgegebenen Zielunterdrucks liegt. Bei aktivierter Saugpumpe 6 wird eine Druckänderungsrate (ΔP/t) ermittelt, und es wird geprüft, ob die Druckänderungsrate in Richtung zunehmenden Unterdrucks innerhalb einer vorgegebenen ersten Zeitspanne Δt1 unterhalb eines vorgegebenen ersten Schwellenwerts S1 bleibt (Prüfung a)).

Wenn dies zutrifft, wird geprüft, ob ein Unterdruck oberhalb eines vorgegebenen zweiten Schwellenwerts S2 vorliegt (Prüfung b)). Wenn dies auch zutrifft, also wenn a) und b) zutreffen, wird die Saugpumpe 6 für eine vorgegebene Zeitdauer von beispielsweise 5 s bis 30 s, vorzugsweise 10 s bis 20 s, insbesondere 15 s, gestoppt und die Druckänderungsrate in Richtung abnehmenden Unterdrucks ermittelt und geprüft, ob die Druckänderungsrate bei ausgeschalteter Saugpumpe oberhalb eines vorgegebenen dritten Schwellenwerts S3 liegt (Prüfung c)). Wenn dies zutrifft, also der Unterdruck rascher abnimmt als S3, wird auf eine Leckage im Unterdrucktherapiesystem geschlossen.

Wenn dies nicht zutrifft, die Druckänderungsrate also nicht oberhalb des vorgegebenen dritten Schwellenwerts S3 liegt, wird geprüft, ob die Druckänderungsrate unterhalb eines vorgegeben vierten Schwellenwerts S4 liegt (Prüfung d)). Wenn dies zutrifft, der Unterdruck also langsamer abnimmt als S4, wird auf eine Blockade im Unterdrucktherapiesystem geschlossen.

Das Schließen auf eine Leckage oder eine Blockade in Abhängigkeit der Druckänderungsrate funktioniert hinreichend zuverlässig, wenn bereits ein bestimmter Unterdruck vorhanden ist. Für den Fall, dass kein Unterdruck oberhalb des vorgegebenen zweiten Schwellenwerts S2 vorliegt (Prüfung b)), erfolgt die Beurteilung von Leckagen oder Blockaden vorzugsweise auf andere Weise. Es wird dann eine elektrische Leistung oder Leistungsaufnahme des Unterdrucktherapiesystems, insbesondere eine elektrische Leistung oder Leistungsaufnahme der Saugpumpe, ermittelt. Zu diesem Zweck umfasst das Unterdrucktherapiesystem 2 in den Figuren nicht weiter dargestellte entsprechende Messeinrichtungen für hierfür erforderliche physikalische Größen, wie insbesondere Strom I, Spannung, sowie beispielsweise geeichte Messwiderstände R.

Weiter wird geprüft, ob die ermittelte elektrische Leistung oder Leistungsaufnahme unterhalb eines vorgegebenen fünften Schwellenwerts S5 liegt (Prüfung e)). Wenn dies zutrifft, also eine geringere Leistung als S5 vorliegt, wird auf eine Leckage geschlossen. Wenn dies nicht zutrifft, also eine höhere Leistung als S5 vorliegt, wird auf eine Blockade geschlossen.

Die einzelnen Verfahrensschritte können, insbesondere zur Verifikation der Prüfergebnisse, einmal oder mehrere Male erneut vorgenommen werden, bevor definitiv auf eine Blockade oder eine Leckage geschlossen wird.

Wenn auf eine Blockade und/oder eine Leckage geschlossen wird, so kann über die Anzeigeelemente 24 des Unterdrucktherapiesystems 2 ein, insbesondere akustisches oder visuelles, Signal ausgegeben werden.

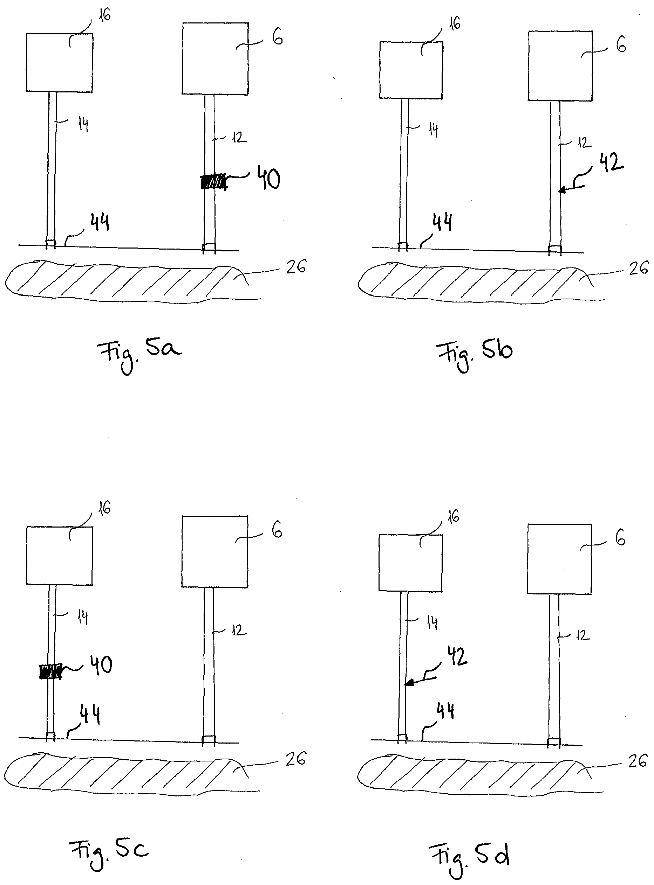

Die Figuren 5a bis 5e zeigen skizzenhaft mögliche Fehlerzustände, die von dem vorausgehend beschriebenen oder einem ähnlichen Unterdrucktherapiesystem 2 durch das Verfahren erfasst werden können, wobei für entsprechende Bauelemente entsprechende Bezugszeichen verwendet werden. In den Figuren 5a bis 5e sind jedoch nur die für die nachfolgende Beschreibung relevanten Komponenten dargestellt. Man erkennt schematisch eine lediglich angedeutete mit Unterdruck zu therapierende Wunde 26 mit einer unterdruckdichten Wundauflage 44, zu der die Saugleitung 12 führt. Über die Saugleitung 12 kommuniziert die Saugpumpe 6 mit der Wunde oder der Wundumgebung, so dass der Wundraum mit Unterdruck beaufschlagbar ist. Ferner führt die Druckmessleitung 14 zur Wunde 26 bzw. zur Wundauflage. In den Figuren 5a bis 5e sind entweder in der Druckmessleitung 14 oder in der Saugleitung 12 oder an der Wundauflage 44 eine Blockade 40 oder eine Leckage 42 eingezeichnet.

Eine Blockade 40 tritt beispielsweise dann auf, wenn die Saugleitung 12 oder die Druckmessleitung 14 verstopft sind oder wenn eine der Leitungen, beispielsweise durch äußere Einwirkungen, abgedrückt oder abgeklemmt ist. Eine Leckage 42 tritt beispielsweise dann auf, wenn eine der Leitungen 12, 14 beschädigt ist oder nicht mehr ordnungsgemäß mit einem Anschluss an der Wundauflage oder am Gehäuse 4 des Unterdrucktherapiesystems 2 verbunden ist.

Der bereits erwähnte Behälter 8 ist in Figuren 5a bis 5e nicht weiter dargestellt. Es ist aber insbesondere denkbar, dass der Behälter 8 zwischen der Saugpumpe 6 und der Saugleitung 12 angeordnet ist, so dass der Behälter 8 mit der Saugpumpe 6 kommuniziert und von dieser mit Unterdruck beaufschlagbar ist und die Saugleitung 12 von dem Behälter 8 aus zur Wunde 26 führt.

In Figur 5a ist skizzenhaft eine Blockade 40 in der Saugleitung 12 dargestellt. Die Blockade 40 kann vor der Inbetriebnahme des Systems vorliegen (Szenario 1) oder erst im laufenden Betrieb auftreten (Szenario 2).

Im Falle des Szenarios 1 wird zunächst mittels des Drucksensors 16 der Druck erfasst und die Saugpumpe 6 aktiviert um einen vorgegebenen Zielunterdruck zu erreichen. Im vorliegenden Fall wird davon ausgegangen, dass der erfasste Druck unterhalb des vorgegebenen Zielunterdrucks liegt. Bei aktivierter Saugpumpe 6 wird die Druckänderungsrate (ΔP/t) ermittelt, und es wird geprüft, ob die Druckänderungsrate in Richtung zunehmenden Unterdrucks unterhalb eines vorgegebenen ersten Schwellenwerts S1 bleibt (Prüfung a)). Im vorliegenden Fall baut sich durch die Blockade 40 in der Saugleitung der Unterdruck an der Wunde 26 und in der Druckmessleitung 14 überhaupt nicht oder nur sehr langsam auf. Die ermittelte Druckänderungsrate liegt daher unter dem Schwellenwert S1. Weiter wird nun geprüft, ob ein Unterdruck oberhalb eines vorgegebenen zweiten Schwellenwerts S2 vorliegt (Prüfung b)). Da die Blockade schon bei Inbetriebnahme vorlag, wird hier davon ausgegangen, dass sich kein oder nur ein sehr geringer Unterdruck aufgebaut hat, so dass der Druck unterhalb des Schwellenwerts S2 liegt (Prüfung b)). Weiter wird nun die elektrische Leistung bzw. Leistungsaufnahme ermittelt und geprüft, ob die elektrische Leistung bzw. Leistungsaufnahme oberhalb oder unterhalb des Schwellwerts S5 liegt. Durch die Blockade 40 in der Saugleitung 12 liegt die elektrische Leistung bzw. Leistungsaufnahme oberhalb des Schwellenwerts S5 (Prüfung e)). Es wird daher auf eine Blockade geschlossen.

Ein möglicher Ablauf des Verfahrens im Falle des Szenarios 2, bei dem die Blockade 40 erst im laufenden Betrieb auftritt, wird nachfolgend beschrieben. Bei aktivierter Saugpumpe 6 wird die Druckänderungsrate (ΔP/t) ermittelt, und es wird geprüft, ob die Druckänderungsrate in Richtung zunehmenden Unterdrucks unterhalb eines vorgegebenen ersten Schwellenwerts S1 bleibt (Prüfung a)). Im vorliegenden Fall wird davon ausgegangen, dass sich durch die Blockade 40 in der Saugleitung an der Wunde 26 und in der Druckmessleitung 14 der Unterdruck überhaupt nicht oder nur sehr langsam ändert. Die ermittelte Druckänderungsrate liegt daher unter dem Schwellenwert S1. Weiter wird nun geprüft, ob ein Unterdruck oberhalb eines vorgegebenen zweiten Schwellenwerts S2 vorliegt (Prüfung b)). In diesem Fall wird davon ausgegangen, dass, da das System bereits in Betrieb war bevor die Blockade 40 aufgetreten ist, Unterdruck oberhalb des Schwellenwerts S2 vorhanden ist (Prüfung b)). Da die Prüfungen a) und b) zutreffen, wird die Saugpumpe 6 für eine vorgegebene Zeitdauer gestoppt und die Druckänderungsrate in Richtung abnehmenden Unterdrucks ermittelt und geprüft, ob eine Druckänderungsrate oberhalb des Schwellenwerts S3 und/oder unterhalb des Schwellenwerts S4 vorliegt. Aufgrund der Blockade 40 wird im vorliegenden Fall davon ausgegangen, dass der Unterdruck gar nicht oder nur sehr langsam abnimmt, zumindest aber langsamer abnimmt als der Schwellenwert S4 (Prüfung d)). Es wird daher auf eine Blockade 40 im Unterdrucktherapiesystem geschlossen.

Sollte im Falle des Szenarios 2, der Druck bereits unterhalb des Schwellwerts S2 gefallen sein (Prüfung b)), beispielsweise da die Blockade 40 bereits über einen längeren Zeitraum vorlag, und der Unterdruck beispielsweise über die Wundauflage 44, die typischer Weise eine gewisse Nachgiebigkeit aufweist, entwichen ist, verläuft Szenario 2 wie Szenario 1.

In Figur 5b ist skizzenhaft eine Leckage 42 in der Saugleitung 12 dargestellt. Die Leckage kann vor der Inbetriebnahme des Systems vorliegen (Szenario 3) oder erst im laufenden Betrieb auftreten (Szenario 4).

Das Szenario 3 kann folgendermaßen ablaufen. Zunächst wird die Saugpumpe 6 aktiviert, um einen vorgegebenen Zielunterdruck zu erreichen. Bei aktivierter Saugpumpe 6 wird die Druckänderungsrate (ΔP/t) ermittelt, und es wird geprüft, ob die Druckänderungsrate in Richtung zunehmenden Unterdrucks unterhalb eines vorgegebenen ersten Schwellenwerts S1 bleibt (Prüfung a)). Im vorliegenden Fall baut sich durch die Leckage 42 in der Saugleitung der Unterdruck überhaupt nicht oder nur sehr langsam auf. Die ermittelte Druckänderungsrate liegt daher unter dem Schwellenwert S1. Weiter wird nun geprüft, ob ein Unterdruck oberhalb eines vorgegebenen zweiten Schwellenwerts S2 vorliegt (Prüfung b)). Da die Leckage 42 schon bei Inbetriebnahme vorlag, wird hier davon ausgegangen, dass sich kein oder nur ein sehr geringer Unterdruck aufgebaut hat, so dass der Druck unterhalb des Schwellenwerts S2 liegt (Prüfung b)). Weiter wird nun die elektrische Leistung bzw. Leistungsaufnahme ermittelt und geprüft, ob die elektrische Leistung bzw. Leistungsaufnahme oberhalb oder unterhalb des Schwellwerts S5 liegt. Durch die Leckage 42 in der Saugleitung 12 liegt die elektrische Leistung bzw. Leistungsaufnahme unterhalb des Schwellenwerts S5 (Prüfung e)). Es wird daher auf eine Leckage geschlossen.

Ein möglicher Ablauf des Verfahrens für das Szenario 4, bei dem die Leckage 42 erst im laufenden Betrieb auftritt, ist nachfolgend beschrieben. Bei aktivierter Saugpumpe 6 wird die Druckänderungsrate (ΔP/t) ermittelt, und es wird geprüft, ob die Druckänderungsrate in Richtung zunehmenden Unterdrucks unterhalb eines vorgegebenen ersten Schwellenwerts S1 bleibt (Prüfung a)). Im vorliegenden Fall wird davon ausgegangen, dass sich durch die Leckage 42 in der Saugleitung 12 der Unterdruck in Richtung zunehmender Unterdruck überhaupt nicht oder nur sehr langsam ändert, oder sogar abnimmt. Die ermittelte Druckänderungsrate liegt daher unter dem Schwellenwert S1. Weiter wird nun geprüft, ob ein Unterdruck oberhalb eines vorgegebenen zweiten Schwellenwerts S2 vorliegt (Prüfung b)). In diesem Fall wird davon ausgegangen, dass durch die Leckage 42 in der Saugleitung 12 überhaupt kein Unterdruck oder nur ein sehr geringer Unterdruck vorhanden ist, also dass der Druck unterhalb des Schwellenwerts S2 liegt (Prüfung b)). Daher wird auch hier die elektrische Leistung bzw. Leistungsaufnahme ermittelt und das Szenario 4 nimmt den weiteren Verlauf des Szenarios 3 an.

In Figur 5c ist skizzenhaft eine Blockade 40 in der Druckmessleitung 14 dargestellt. Die Blockade 40 kann vor Inbetriebnahme des Systems vorliegen (Szenario 5) oder erst im laufenden Betrieb auftreten (Szenario 6).

Im Falle des Szenarios 5 wird zunächst die Saugpumpe 6 aktiviert um einen vorgegebenen Zielunterdruck zu erreichen. Im vorliegenden Fall wird davon ausgegangen, dass sich in der Saugleitung und im Wundraum Unterdruck aufbaut. Durch die Blockade 40 in der Druckmessleitung bleibt der Druck in der Druckmessleitung jedoch nahezu unverändert. Die ermittelte Druckänderungsrate liegt daher unter dem Schwellenwert S1 (Prüfung a)). Weiter wird nun geprüft, ob ein Unterdruck oberhalb eines vorgegebenen zweiten Schwellenwerts S2 vorliegt (Prüfung b)). Da die Blockade 40 schon bei Inbetriebnahme vorlag, wird hier davon ausgegangen, dass in der Druckmessleitung 14 kein Unterdruck vorliegt und der erfasste Druck unterhalb des Schwellenwerts S2 liegt (Prüfung b)). Weiter wird nun die elektrische Leistung bzw. Leistungsaufnahme ermittelt und geprüft, ob die elektrische Leistung bzw. Leistungsaufnahme oberhalb oder unterhalb des Schwellwerts S5 liegt (Prüfung e)).

Durch die Blockade 40 in der Druckmessleitung 14 liegt die elektrische Leistung bzw. Leistungsaufnahme oberhalb des Schwellenwerts S5 (Prüfung e)). Es wird daher auf eine Blockade geschlossen.

Ein möglicher Ablauf des Verfahrens für Szenario 6, bei dem die Blockade 40 in der Druckmessleitung 14 erst im Betrieb auftritt, ist nachfolgend beschrieben. Bei aktivierter Saugpumpe 6 wird die Druckänderungsrate (ΔP/t) ermittelt, und es wird geprüft, ob die Druckänderungsrate in Richtung zunehmenden Unterdrucks unterhalb eines vorgegebenen ersten Schwellenwerts S1 bleibt (Prüfung a)). Im vorliegenden Fall wird davon ausgegangen, dass durch die Blockade 40 in der Druckmessleitung 14 der Druck in der Druckmessleitung 14 jedoch nahezu unverändert bleibt und die ermittelte Druckänderungsrate daher unter dem Schwellenwert S1 liegt. Weiter wird nun geprüft, ob ein Unterdruck oberhalb eines vorgegebenen zweiten Schwellenwerts S2 vorliegt (Prüfung b)).

In diesem Fall wird davon ausgegangen, dass, da das System bereits in Betrieb war bevor die Blockade 40 aufgetreten ist, Unterdruck in der Druckmessleitung 14 vorhanden ist, der zumindest oberhalb des Schwellenwerts S2 liegt. Da die Prüfungen a) und b) zutreffen, wird die Saugpumpe 6 für eine vorgegebene Zeitdauer gestoppt und die Druckänderungsrate in Richtung abnehmenden Unterdrucks ermittelt. Aufgrund der Blockade 40 in der Druckmessleitung 14 wird im vorliegenden Fall davon ausgegangen, dass der Unterdruck bei deaktivierter Saugpumpe 6 in der Druckmessleitung 14 gar nicht oder nur sehr langsam abnimmt, zumindest aber langsamer abnimmt als der Schwellenwert S4 (Prüfung d)). Es wird daher auf eine Blockade 40 im Unterdrucktherapiesystem geschlossen.

In Figur 5d ist skizzenhaft eine Leckage 42 in der Druckmessleitung 14 dargestellt. Die Leckage 42 kann vor der Inbetriebnahme des Systems vorliegen (Szenario 7) oder erst im laufenden Betrieb auftreten (Szenario 8).

Im Falle des Szenarios 7 läuft das Verfahren wie nachfolgend beschrieben ab. Zunächst wird die Saugpumpe 6 aktiviert um einen vorgegebenen Zielunterdruck zu erreichen. Bei aktivierter Saugpumpe 6 wird die Druckänderungsrate (ΔP/t) ermittelt, und es wird geprüft, ob die Druckänderungsrate in Richtung zunehmenden Unterdrucks unterhalb eines vorgegebenen ersten Schwellenwerts S1 bleibt (Prüfung a)). Im vorliegenden Fall baut sich durch die Leckage 42 in der Druckmessleitung 14 der Unterdruck zumindest in der Druckmessleitung 14 überhaupt nicht oder nur sehr langsam auf. Die ermittelte Druckänderungsrate liegt daher unter dem Schwellenwert S1. Weiter wird nun geprüft, ob ein Unterdruck oberhalb eines vorgegebenen zweiten Schwellenwerts S2 vorliegt (Prüfung b)). Da die Leckage 42 schon bei Inbetriebnahme vorlag, wird hier davon ausgegangen, dass kein oder nur ein sehr geringer Unterdruck vorliegt, so dass der Druck unterhalb des Schwellenwerts S2 liegt (Prüfung b)). Weiter wird nun die elektrische Leistung bzw. Leistungsaufnahme ermittelt und geprüft, ob die elektrische Leistung bzw. Leistungsaufnahme oberhalb oder unterhalb des Schwellwerts S5 liegt. Durch die Leckage 42 in der Druckmessleitung 14 liegt die elektrische Leistung bzw. Leistungsaufnahme unterhalb des Schwellenwerts S5 (Prüfung e)). Es wird daher auf eine Leckage geschlossen.

Ein möglicher Ablauf des Verfahrens für Szenario 8, bei dem die Leckage in der Druckmessleitung 14 erst im Betrieb auftritt, ist nachfolgend beschrieben. Bei aktivierter Saugpumpe 6 wird die Druckänderungsrate (ΔP/t) ermittelt, und es wird geprüft, ob die Druckänderungsrate in Richtung zunehmenden Unterdrucks unterhalb eines vorgegebenen ersten Schwellenwerts S1 bleibt (Prüfung a)). Im vorliegenden Fall wird davon ausgegangen, dass sich durch die Leckage 42 in der Druckmessleitung 14 der Unterdruck in Richtung zunehmenden Unterdrucks überhaupt nicht oder nur sehr langsam ändert, oder sogar abnimmt. Die ermittelte Druckänderungsrate liegt daher unter dem Schwellenwert S1. Weiter wird nun geprüft, ob ein Unterdruck oberhalb eines vorgegebenen zweiten Schwellenwerts S2 vorliegt (Prüfung b)). In diesem Fall wird davon ausgegangen, dass durch die Leckage 42 überhaupt kein Unterdruck oder nur ein sehr geringer Unterdruck in der Druckmessleitung 14 vorhanden ist und der erfasste Druck daher unterhalb des Schwellenwerts S2 liegt. Daher wird auch in diesem Fall die elektrische Leistung bzw. Leistungsaufnahme ermittelt und das Szenario 8 nimmt den weiteren Verlauf des Szenarios 7 an.

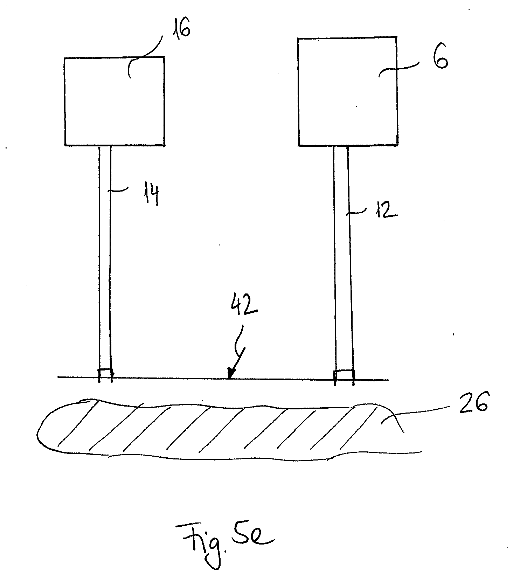

Schließlich ist in Figur 5e eine Leckage 42 an der Wundauflage 44 dargestellt. Im vorliegenden Fall wird davon ausgegangen, dass die Leckage 42 erst im laufenden Betrieb aufgetreten ist (Szenario 9). Bei aktivierter Saugpumpe 6 wird die Druckänderungsrate (ΔP/t) ermittelt, und es wird geprüft, ob die Druckänderungsrate in Richtung zunehmenden Unterdrucks unterhalb eines vorgegebenen ersten Schwellenwerts S1 bleibt (Prüfung a)). Im vorliegenden Fall wird davon ausgegangen, dass sich infolge der Leckage 42 an der Wundauflage der Unterdruck in Richtung zunehmenden Unterdrucks überhaupt nicht oder nur sehr langsam ändert, oder der Unterdruck sogar abnimmt. Die ermittelte Druckänderungsrate in Richtung zunehmenden Unterdrucks wird daher unter dem Schwellenwert S1 liegen. Weiter wird nun geprüft, ob (trotz der Leckage) ein Unterdruck oberhalb eines vorgegebenen zweiten Schwellenwerts S2 vorliegt (Prüfung b)). Wenn die Prüfungen a) und b) zutreffen, wird die Saugpumpe 6 für eine vorgegebene Zeitdauer gestoppt und die Druckänderungsrate in Richtung abnehmenden Unterdrucks ermittelt. Aufgrund der Leckage 42 an der Wundauflage 44 wird im vorliegenden Fall davon ausgegangen, dass bei deaktivierter Saugpumpe der Unterdruck in der Druckmessleitung mit einer Druckänderungsrate oberhalb des Schwellenwerts S3, also rascher als S3, abnimmt (Prüfung c)). Es wird daher auf eine Leckage 42 im Unterdrucktherapiesystem geschlossen werden.

Die hier beschriebenen Szenarien 1 bis 9 sind nur beispielhaft. Es sind darüber hinaus noch weitere Fehlerzustände im Unterdruckmesssystem 2 denkbar. Beispielsweise kann eine Leckage 42 und/oder Blockade 40, insbesondere zeitgleich, auch an mehreren Stellen im Unterdrucktherapiesystem 2 auftreten. Solche und weitere Fehlerzustände werden in ähnlicher Weise durch das beschriebene Verfahren als Leckagen 42 oder Blockaden 40 erkannt.

## Patentansprüche

1. Unterdrucktherapiesystem (2) zum Bereitstellen von Unterdruck zur medizinischen Unterdruckbehandlung von Wunden am menschlichen oder tierischen Körper, umfassend
- eine Unterdruck erzeugende Saugpumpe (6),
- eine zum Körper führende Saugleitung (12), wobei die Saugleitung (12) von der Saugpumpe (6) mit Unterdruck beaufschlagt wird,
- optional einen Behälter (8) zur Aufnahme von Körperflüssigkeiten, insbesondere von aus einer Wunde (26) abgesaugten Wundsekreten,
- einen Drucksensor (16) zum Erfassen des Drucks in einer zum Körper führenden Druckmessleitung (14) oder in der Saugleitung (12) oder im Behälter (8) oder in der Wunde (26),
- eine elektronische Steuereinrichtung (10), welche mindestens unter Berücksichtigung von vorgegebenen und/oder vorgebbaren Parametern und von durch den Drucksensor (16) erfassten Druckwerten die Saugpumpe (6) ansteuert; wobei die elektronische Steuereinrichtung zum Ausführen eines Verfahrens umfassend die folgenden Schritte ausgebildet ist:
Erfassen des Drucks und Aktivieren der Saugpumpe (6) um einen vorgegebenen Zielunterdruck zu erreichen, und Deaktivieren der Saugpumpe (6), wenn der Zielunterdruck vorliegt, **dadurch gekennzeichnet, dass** bei aktivierter Saugpumpe (6) geprüft wird, ob innerhalb einer vorgegebenen ersten Zeitspanne Δt1 eine Druckänderungsrate in Richtung zunehmenden Unterdrucks unterhalb eines vorgegebenen ersten Schwellenwerts S1 bleibt, und dass geprüft wird, ob ein Unterdruck oberhalb eines vorgegebenen zweiten Schwellenwerts S2 vorliegt, und sofern beides zutrifft, die Saugpumpe (6) gestoppt wird, und dass bei gestoppter Saugpumpe (6) die Druckänderungsrate in Richtung abnehmenden Unterdrucks ermittelt wird, und dass auf eine Leckage im Unterdrucktherapiesystem (2) geschlossen wird, wenn die Druckänderungsrate oberhalb eines vorgegebenen dritten Schwellenwerts S3 liegt, und/oder dass auf eine Blockade im Unterdrucktherapiesystem (2) geschlossen wird, wenn die Druckänderungsrate unterhalb eines vorgegebenen vierten Schwellenwerts S4 liegt.

2. Unterdrucktherapiesystem (2) nach Anspruch 1, **dadurch gekennzeichnet, dass** die vorgegebene erste Zeitspanne Δt1 0,5s bis 2s, vorzugsweise 0,8s bis 1,3s, insbesondere 1s, beträgt.

3. Unterdrucktherapiesystem (2) nach einem der Ansprüche 1 oder 2, **dadurch gekennzeichnet, dass** der erste Schwellenwert S1 0 mmHg/s beträgt.

4. Unterdrucktherapiesystem (2) nach einem oder mehreren der vorhergehenden Ansprüche, **dadurch gekennzeichnet, dass** der zweite Schwellenwert S2 10 mmHg bis 25 mmHg, vorzugweise 10 mmHg bis 20 mmHg, insbesondere 15 mmHg, beträgt.

5. Unterdrucktherapiesystem (2) nach einem oder mehreren der vorhergehenden Ansprüche, **dadurch gekennzeichnet, dass** der dritte Schwellenwert S3 5 mmHg/15 s bis 15 mmHg/15 s, insbesondere 8 mmHg/15 s, und/oder der vierte Schwellenwert S4 1 mmHg/15 s bis 5 mmHg/15 s, insbesondere 2 mmHg/15 s, beträgt.

6. Unterdrucktherapiesystem (2) nach einem oder mehreren der vorhergehenden Ansprüche, **dadurch gekennzeichnet, dass** die Saugpumpe (6) für eine Dauer von 5 s bis 30 s, vorzugsweise 10 s bis 20 s, insbesondere 15 s gestoppt wird.

7. Unterdrucktherapiesystem (2) nach einem oder mehreren der vorhergehenden Ansprüche, **dadurch gekennzeichnet, dass** geprüft wird, ob innerhalb der vorgegebenen ersten Zeitspanne Δt1 eine Druckänderungsrate in Richtung zunehmenden Unterdrucks unterhalb des vorgegebenen ersten Schwellenwerts S1 bleibt, und dass geprüft wird, ob ein Unterdruck oberhalb des vorgegebenen zweiten Schwellenwerts S2 vorliegt, und sofern ersteres zutrifft und zweiteres nicht zutrifft, eine elektrische Leistung oder Leistungsaufnahme des Unterdrucktherapiesystems (2) ermittelt wird, und dass auf eine Leckage im Unterdrucktherapiesystem (2) geschlossen wird, wenn die elektrische Leistung oder Leistungsaufnahme unterhalb eines vorgegebenen fünften Schwellenwerts S5 liegt, und/oder dass auf eine Blockade im Unterdrucktherapiesystem (2) geschlossen wird, wenn die elektrische Leistung oder Leistungsaufnahme oberhalb des vorgegebenen fünften Schwellenwerts S5 liegt.

8. Unterdrucktherapiesystem (2) nach einem oder mehreren der vorhergehenden Ansprüche, **dadurch gekennzeichnet, dass** bei Schließen auf eine Leckage und/oder bei Schließen auf eine Blockade, ein Signal ausgegeben wird.

9. Unterdrucktherapiesystem (2) nach einem oder mehreren der vorhergehenden Ansprüche, **dadurch gekennzeichnet, dass** bei Schließen auf eine Leckage und/oder bei Schließen auf eine Blockade die Verfahrensschritte gemäß der Ansprüche 1 und/oder 7 wenigstens einmal oder mehrere Male erneut ausgeführt werden, bevor auf eine Leckage und/oder eine Blockade geschlossen wird.

10. Unterdrucktherapiesystem (2) nach einem oder mehreren der vorhergehenden Ansprüche, **dadurch gekennzeichnet, dass** geprüft wird, ob der vorgegebene Zielunterdruck vorliegt, und dass geprüft wird, ob die Saugpumpe (6) seit mindestens einer vorgegebenen zweiten Zeitspanne Δt2 deaktiviert ist, und sofern beides zutrifft, die Saugpumpe (6) für eine vorgegebene dritte Zeitspanne Δt3 aktiviert wird und anschließend bei deaktivierter Saugpumpe (6) über eine Zeitspanne Δt4 eine maximale Druckänderung in Richtung zunehmenden Unterdrucks und/oder eine maximale Druckänderung in Richtung abnehmenden Unterdrucks ermittelt und geprüft wird, ob die maximale Druckänderung in Richtung zunehmenden Unterdrucks oberhalb eines vorgegebenen sechsten Schwellenwerts S6 und/oder die maximale Druckänderung in Richtung abnehmenden Unterdrucks oberhalb eines vorgegebenen siebten Schwellenwerts S7 liegt.

## Claims

1. Negative pressure therapy system (2) for providing negative pressure for the medical negative pressure treatment of wounds on the human or animal body, comprising
- a suction pump (6) which generates negative pressure,
- a suction line (12) leading to the body, the suction line (12) being subjected to negative pressure by the suction pump (6),
- optionally a container (8) for receiving bodily fluids, in particular from wound secretions suctioned out of a wound (26),
- a pressure sensor (16) for detecting the pressure in a pressure measuring line (14) leading to the body or in the suction line (12) or in the container (8) or in the wound (26),
- an electronic control device (10) which controls the suction pump (6) at least taking into account predefined and/or predefinable parameters and pressure values detected by the pressure sensor (16); the electronic control device being designed to carry out a method comprising the following steps:
detecting the pressure and activating the suction pump (6) in order to achieve a predefined target negative pressure, and deactivating the suction pump (6) when the target negative pressure is present, **characterized in that**, when the suction pump (6) is activated, a check is carried out as to whether a pressure change rate toward increasing negative pressure remains below a predefined first threshold value S1 within a predefined first time span Δt1, and **in that** a check is carried out as to whether a negative pressure above a predefined second threshold value S2 is present, and, if both are true, the suction pump (6) is stopped, and **in that**, when the suction pump (6) is stopped, the pressure change rate toward decreasing negative pressure is determined, and **in that** a leak in the negative pressure therapy system (2) is inferred if the pressure change rate is above a predefined third threshold value S3, and/or **in that** a blockage in the negative pressure therapy system (2) is inferred if the pressure change rate is below a predefined fourth threshold value S4.

2. Negative pressure therapy system (2) according to claim 1, **characterized in that** the predefined first time period Δt1 is 0.5 s to 2 s, preferably 0.8 s to 1.3 s, in particular 1 s.

3. Negative pressure therapy system (2) according to claim 1 or claim 2, **characterized in that** the first threshold value S1 is 0 mmHg/s.

4. Negative pressure therapy system (2) according to one or more of the preceding claims, **characterized in that** the second threshold value S2 is 10 mmHg to 25 mmHg, preferably 10 mmHg to 20 mmHg, in particular 15 mmHg.

5. Negative pressure therapy system (2) according to one or more of the preceding claims, **characterized in that** the third threshold value S3 is 5 mmHg/15 s to 15 mmHg/15 s, in particular 8 mmHg/15 s, and/or the fourth threshold value S4 is 1 mmHg/15 s to 5 mmHg/15 s, in particular 2 mmHg/15 s.

6. Negative pressure therapy system (2) according to one or more of the preceding claims, **characterized in that** the suction pump (6) is stopped for a duration of 5 s to 30 s, preferably 10 s to 20 s, in particular 15 s.

7. Negative pressure therapy system (2) according to one or more of the preceding claims, **characterized in that** a check is carried out as to whether a pressure change rate toward increasing negative pressure remains below the predefined first threshold value S1 within the predefined first time period Δt1, and **in that** a check is carried out as to whether a negative pressure above the predefined second threshold value S2 is present, and, if the first is true but the second is not true, an electrical power or power consumption of the negative pressure therapy system (2) is determined, and **in that** a leak in the negative pressure therapy system (2) is inferred if the electrical power or power consumption is below a predefined fifth threshold value S5, and/or **in that** a blockage in the negative pressure therapy system (2) is inferred if the electrical power or power consumption is above the predefined fifth threshold value S5.

8. Negative pressure therapy system (2) according to one or more of the preceding claims, **characterized in that** a signal is output when a leak is inferred and/or when a blockage is inferred.

9. Negative pressure therapy system (2) according to one or more of the preceding claims, **characterized in that**, when a leak is inferred and/or when a blockage is inferred, the method steps according to claims 1 and/or 7 are carried out again at least once or several times before a leak and/or a blockage is inferred.

10. Negative pressure therapy system (2) according to one or more of the preceding claims, **characterized in that** a check is carried out as to whether the predefined target negative pressure is present, and **in that** a check is carried out as to whether the suction pump (6) is deactivated for at least a predefined second time period Δt2, and, if both are true, the suction pump (6) is activated for a predefined third period of time Δt3 and then, when the suction pump (6) is deactivated over a period of time Δt4, a maximum pressure change toward increasing negative pressure and/or a maximum pressure change toward decreasing negative pressure is determined and a check is carried out as to whether the maximum pressure change toward increasing negative pressure is above a predefined sixth threshold value S6 and/or the maximum pressure change toward decreasing negative pressure is above a predefined seventh threshold value S7.

## Revendications

1. Système de thérapie par pression négative (2) destiné à fournir de la pression négative pour le traitement médical par pression négative de plaies sur le corps humain ou animal, comprenant
- une pompe d'aspiration (6) générant de la pression négative,
- une conduite d'aspiration (12) menant vers le corps, la conduite d'aspiration (12) étant soumise à une pression négative par la pompe d'aspiration (6),
- en option un récipient (8) destiné à recevoir des fluides corporels, en particulier des sécrétions de plaie aspirées d'une plaie (26),
- un capteur de pression (16) destiné à détecter la pression à l'intérieur d'une conduite de mesure de pression (14) menant vers le corps ou à l'intérieur de la conduite d'aspiration (12) ou à l'intérieur du récipient (8) ou dans la plaie (26),
- un dispositif de commande électronique (10) qui commande la pompe d'aspiration (6) au moins en tenant compte de paramètres prédéterminés et/ou aptes à être prédéterminés et des valeurs de pression détectées par le capteur de pression (16); dans lequel ledit dispositif de commande électronique est conçu pour mettre en œuvre un procédé comprenant les étapes suivantes consistant à:
détecter la pression et activer la pompe d'aspiration (6) afin d'atteindre une pression négative cible prédéterminée, et désactiver la pompe d'aspiration (6) lorsque la pression négative cible est présente, **caractérisé par le fait que**, lorsque la pompe d'aspiration (6) est activée, il est vérifié si, dans un premier intervalle de temps Δt1 prédéterminé, un taux de changement de pression dans le sens d'une augmentation de la pression négative reste inférieur à une première valeur de seuil S1 prédéterminée, et qu'il est vérifié s'il y a une pression négative supérieure à une deuxième valeur de seuil S2 prédéterminée, et si les deux cas sont vrais,la pompe d'aspiration (6) est arrêtée, et que, lorsque la pompe d'aspiration (6) est arrêtée, le taux de changement de pression dans le sens d'une diminution de la pression négative est déterminé, et qu'il est conclu à une fuite dans le système de thérapie par pression négative (2) si le taux de changement de pression est supérieur à une troisième valeur de seuil S3 prédéterminée, et/ou qu'il est conclu à un blocage dans le système de thérapie par pression négative (2) si le taux de changement de pression est inférieur à une quatrième valeur de seuil S4 prédéterminée.

2. Système de thérapie par pression négative (2) selon la revendication 1, **caractérisé par le fait que** le premier intervalle de temps Δt1 prédéterminé est compris entre 0,5 s et 2 s, de préférence entre 0,8 s et 1,3 s, en particulier il est de 1s.

3. Système de thérapie par pression négative (2) selon l'une quelconque des revendications 1 ou 2, **caractérisé par le fait que** la première valeur de seuil S1 est de 0 mmHg/s.

4. Système de thérapie par pression négative (2) selon une ou plusieurs des revendications précédentes, **caractérisé par le fait que** la deuxième valeur de seuil S2 est comprise entre 10 mmHg et 25 mmHg, de préférence entre 10 mmHg et 20 mmHg, en particulier elle est de 15 mmHg.

5. Système de thérapie par pression négative (2) selon une ou plusieurs des revendications précédentes, **caractérisé par le fait que** la troisième valeur de seuil S3 est comprise entre 5 mmHg/15 s et 15 mmHg/15 s, en particulier elle est de 8 mmHg/15 s, et/ou la quatrième valeur de seuil S4 est comprise entre 1 mmHg/15 s et 5 mmHg/15 s, en particulier elle est de 2 mmHg/15 s.

6. Système de thérapie par pression négative (2) selon une ou plusieurs des revendications précédentes, **caractérisé par le fait que** la pompe d'aspiration (6) est arrêtée pendant une durée de 5 s à 30 s, de préférence de 10 s à 20 s, en particulier de 15 s.

7. Système de thérapie par pression négative (2) selon une ou plusieurs des revendications précédentes, **caractérisé par le fait qu'**on vérifie si, dans le premier intervalle de temps Δt1 prédéterminé, un taux de changement de pression dans le sens d'une augmentation de la pression négative reste inférieur à la première valeur de seuil S1 prédéterminée, et qu'on vérifie si une pression négative supérieure à la deuxième valeur de seuil S2 prédéterminée est présente, et si le premier cas est vrai et le deuxième cas n'est pas vrai, une puissance électrique ou une puissance absorbée du système de thérapie par pression négative (2) est déterminée, et qu'il est conclu à une fuite dans le système de thérapie par pression négative (2) si la puissance électrique ou la puissance absorbée est inférieure à une cinquième valeur de seuil S5 prédéterminée, et/ou qu'il est conclu à un blocage dans le système de thérapie par pression négative (2) si la puissance électrique ou la puissance absorbée est supérieure à la cinquième valeur de seuil S5 prédéterminée.

8. Système de thérapie par pression négative (2) selon une ou plusieurs des revendications précédentes, **caractérisé par le fait qu'**un signal est émis lorsqu'il est conclu à une fuite et/ou lorsqu'il est conclu à un blocage.

9. Système de thérapie par pression négative (2) selon une ou plusieurs des revendications précédentes, **caractérisé par le fait que**, lorsqu'il est conclu à une fuite et/ou lorsqu'il est conclu à un blocage, les étapes de procédé selon les revendications 1 et/ou 7 sont mises en œuvre au moins une fois ou plusieurs fois avant de conclure à une fuite et/ou à un blocage.

10. Système de thérapie par pression négative (2) selon une ou plusieurs des revendications précédentes, **caractérisé par le fait qu'**on vérifie si la pression négative cible prédéterminée est présente, et qu'on vérifie si la pompe d'aspiration (6) est désactivée depuis au moins un deuxième intervalle de temps Δt2 prédéterminé, et si les deux cas sont vrais, la pompe d'aspiration (6) est activée pendant un troisième intervalle de temps Δt3 prédéterminé, et ensuite, avec la pompe d'aspiration (6) désactivée, un changement de pression maximal dans le sens d'une augmentation de la pression négative et/ou un changement de pression maximal dans le sens d'une diminution de la pression négative est déterminé pendant un intervalle de temps Δt4, et qu'on vérifie si le changement de pression maximal dans le sens d'une augmentation de la pression négative est supérieure à une sixième valeur de seuil S6 prédéterminée et/ou si le changement de pression maximal dans le sens d'une diminution de la pression négative est supérieure à une septième valeur de seuil S7 prédéterminée.
